# EUROPEAN PATENT APPLICATION

(11) **EP 2 641 609 A1**
(43) Date of publication of application: **25.09.2013**
(21) Application number: 11841477.0
(22) Date of filing: 17.11.2011
(51) Int. Cl.: A61K 38/00, A61P 21/00

(54) **THERAPEUTIC AGENT FOR MUSCULAR DYSTROPHY**

(30) Priority: 17.11.2010 JP 2010256873
(71) Applicant: Fukuda, Keiichi, Tokyo 1760006 (JP)
(72) Inventor: FUKUDA, Keiichi., Tokyo 176-0006, (JP); YUASA, Shinsuke., Tokyo 145-0071 (JP)
(74) Representative: Vossius & Partner
(86) International application number: PCT/JP2011/077113
(87) International publication number: WO 2012/067262

(57) **Abstract**

The present invention provides therapeutic agents for diseases associated with skeletal muscle degeneration comprising a granulocyte colony-stimulating factor (G-CSF) as an active ingredient. The therapeutic agents of the present invention are especially effective as therapeutic agents for muscular dystrophy.

SEQUENCE LISTING
<110> Fukuda, Keiichi
<120> Therapeutic agents for muscular dystrophy
<130> FA0001-11102

<150> JP2010-256873
<151> 2010-11-17

<160> 9

<210> 1
<211> 27
<212> DNA
<213> Artificial
<400> 1
catagttatt aatgcataga tattcag 27

<210> 2
<211> 27
<212> DNA
<213> Artificial
<400> 2
gtcactcaga tagttgaagc catttag 27

<210> 3
<211> 27
<212> DNA
<213> Artificial
<400> 3
gtcactcaga tagttgaagc catttaa 27

<210> 4
<211> 19
<212> DNA
<213> Artificial
<400> 4
gtgaaggatg tcatgaaag 19

<210> 5
<211> 20
<212> DNA
<213> Artificial
<400> 5
tgaagtccga aagagatacc 20

<210> 6
<211> 20
<212> DNA
<213> Artificial
<400> 6
acgagactag tgagacgtgc 20

<210> 7
<211> 20
<212> DNA
<213> Artificial
<400> 7
agttcaccag gcaggtgagt 20

<210> 8
<211> 20
<212> DNA
<213> Artificial
<400> 8
gtaggcctag ttcatacctg 20

<210> 9
<211> 20
<212> DNA
<213> Artificial
<400> 9
tccagactgc cttgggaaaa 20

## Description

### TECHNICAL FIELD

The present invention relates to therapeutic agents for diseases associated with skeletal muscle degeneration, especially therapeutic agents for muscular dystrophy. The therapeutic agents for diseases associated with skeletal muscle degeneration of the present invention comprise a granulocyte colony-stimulating factor (G-CSF) as an active ingredient.

### BACKGROUND ART

Muscular dystrophy collectively refers to inheritable diseases characterized by main symptoms such as progressive muscle weakness and muscular atrophy caused by degeneration and necrosis of myofiber and currently classified by their mode of inheritance and clinical features into ten or more types including Duchenne, Becker, limb-girdle, congenital, distal, facioscapulohumeral, myotonic, etc. Among them, Duchenne patients are the most common, and previous studies have been focused on Duchenne type.

Duchenne muscular dystrophy (DMD) is inherited in an X-linked recessive pattern and predominately affects males. It is said to affect one in 3,500 male newborns. The patients show symptoms of muscle weakness during childhood, and once the disease develops, muscular atrophy proceeds relentlessly. Muscle tissue is gradually replaced by fat and fibrous tissue, and the patients lose the ability to walk at around 10 years of age, and die of progressive weakness, respiratory failure or heart failure at around 20 years of age on average.

It has been shown that DMD is caused by abnormality (deletion or mutation or the like) of the dystrophin gene located in the short arm of the X chromosome (Xp21) (non-patent document 1). The Dystrophin protein (Mw 427 kD), that is the gene product of the patent document 1). The Dystrophin protein (Mw 427 kD), that is the gene product of the dystrophin gene, is localized just beneath the plasma membrane of myocytes and functions to maintain the cytoskeleton of myocytes by binding intracellularly to actin among proteins of which myofibrils are composed and also binding to laminin in the extracellular matrix through the dystrophin-associated glycoprotein complex at the plasma membrane (non-patent document 2). In DMD patients, no dystrophin exists in the myocyte membrane because normal dystrophin is not produced due to abnormality in the dystrophin gene. It is thought that this may be why myocytes cannot retain their shape and are more susceptible to necrosis by injury during contraction or the like.

At present, any effective therapy for DMD has not been found yet, and importance is placed on rehabilitation for retarding the progression of symptoms or respiratory management using mechanical ventilators or the like. Drug therapy includes corticosteroids (steroids), but they have strong side effects and have not produced sufficient therapeutic effect. Experimental therapies such as regenerative therapy (transplantation of stem cells and myoblasts) (non-patent document 3), gene therapy (functional dystrophin gene transfer, antisense morpholino-mediated skipping of mutated exons) (non-patent document 4), new drug therapy (read-through of nonsense mutations) (non-patent document 5) and the like have been tried, but have not been practically applied. Moreover, any immunosuppressants have not actually shown reliable effect except for glucocorticoids, which in turn have not been explained about their muscle strengthening mechanism and which should be further studied to verify their effects.

Thus, a drug acting suppressively on myofiber disruption in muscular dystrophy would be expected to be effective for treating or retarding the progression of muscular dystrophy.

### CITATION LIST

Non-patent document 1: Koenig, M. et al. Complete cloning of the Duchenne muscular dystrophy (DMD) cDNA and preliminary genomic organization of the DMD gene in normal and affected individuals. Cell 50, 509-517 (1987).
Non-patent document 2: Matsumura, K. & Campbell, K. P. Dystrophin-glycoprotein complex: its role in the molecular pathogenesis of muscular dystrophies. Muscle Nerve 17, 2-15 (1994).
Non-patent document 3: Meregalli, M. et al. Stem cell therapies to treat muscular dystrophy: progress to date. BioDrugs 24, 237-247 (2010).
Non-patent document 4: Kinali, M. et al. Local restoration of dystrophin expression with the morpholino oligomer AVI-4658 in Duchenne muscular dystrophy: a single-blind, placebo-controlled, dose-escalation, proof-of-concept study. Lancet Neurol. 8, 918-928 (2009).
Non-patent document 5: Finkel, R. S. Read-through strategies for suppression of nonsense mutations in Duchenne/ Becker muscular dystrophy: aminoglycosides and ataluren (PTC124). J. Child Neurol. 25, 1158-1164 (2010).

### SUMMARY OF INVENTION

### TECHNICAL PROBLEM

The present invention aims to provide a therapeutic agent for a disease associated with skeletal muscle degeneration comprising a G-CSF, especially a therapeutic agent for muscular dystrophy. Specifically, it aims to elucidate the effect of G-CSF on injured muscles and the underlying mechanism and further to determine the route and frequency of administration and dose of G-CSF suitable for use as a therapeutic agent for muscular dystrophy, thereby providing a therapeutic agent that can be used practically for treating muscular dystrophy.

### SOLUTION TO PROBLEM

We studied the effect of G-CSF on degenerated muscles and the underlying mechanism. As a result, we found that muscle degeneration occurs at postnatal week 3-5 and the G-CSF receptor (G-CSFR) is expressed specifically in regenerated muscles at this stage in *mdx* mice known as one of DMD model mice and that significant muscle regeneration was observed when G-CSF was administered. Further, not only significant muscle regeneration but also long-term survival up to 200 days was observed when G-CSF was administered to *mdx*/*utrn*^{-/-} mice known as DMD model mice showing severe symptoms similar to human DMD. The present invention was accomplished on the basis of these findings.

Accordingly, the present invention provides the following.
[1] A therapeutic agent for a disease associated with skeletal muscle degeneration comprising a granulocyte colony-stimulating factor (G-CSF) as an active ingredient.
[2] The therapeutic agent of [1] wherein the disease associated with skeletal muscle degeneration is muscular dystrophy.
[3] The therapeutic agent of [2] wherein the disease associated with skeletal muscle degeneration is Duchenne muscular dystrophy.

### ADVANTAGEOUS EFFECTS OF INVENTION

The therapeutic agents for diseases associated with skeletal muscle degeneration of the present invention were shown to be especially effective as therapeutic agents for muscular dystrophy and to act suppressively on myofiber disruption in muscular dystrophy. They can be expected to be used for treating muscular dystrophy that is an intractable disease for which no efficacious therapy has been available so far.

### BRIEF DESCRIPTION OF DRAWINGS

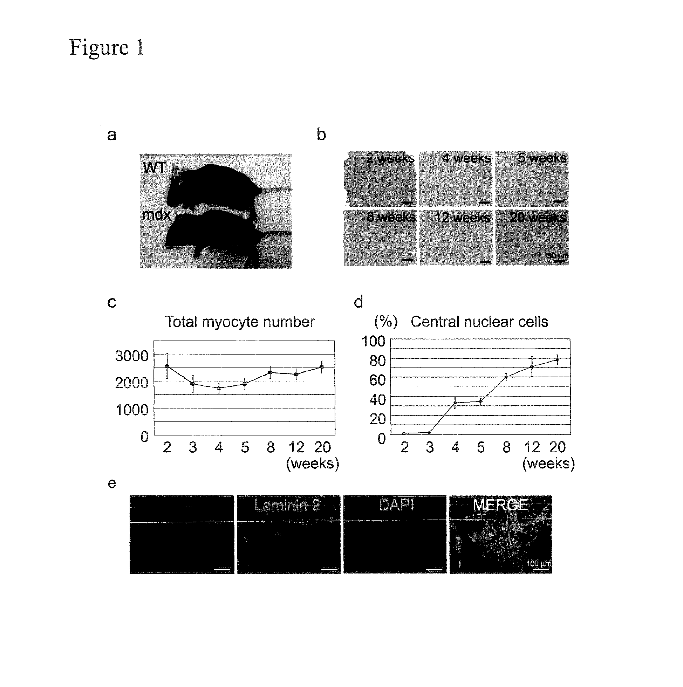

[Figure 1]
   a. A comparison of the gross appearances of wild-type mice (WT) and *mdx* mice (mdx) known as muscular dystrophy model mice is shown. Unlike human DMD, no physical abnormality is observed in *mdx* mice.
   b. Images of H&E stained sections of tibialis anterior (TA) muscles from *mdx* mice at 2-20 weeks of age are shown. Centrally nucleated cells (regenerated muscles) increase at 4-5 weeks of age, showing that muscles are repeatedly injured and regenerated at this stage.
   c. The total number of myocytes in TA muscle sections from *mdx* mice is shown. It is observed that the total number of the cells decreases at 2-4 weeks of age and then recovers.
   d. The proportion of centrally nucleated myocytes to the total number of myocytes in *mdx* mice is shown.
   e. Images of TA muscle sections from *mdx* mice fluorescently stained with an anti-G-CSFR antibody, an anti-laminin-2α antibody and DAPI, respectively, and an image formed by superimposing these images (MERGE) are shown. It is observed that G-CSFR is expressed specifically in cells having nuclei stained with DAPI (regenerating muscles).
[Figure 2]
   a. A schedule of G-CSF administration to *mdx* mice is shown. G-CSF is administered at the stage during which muscles are acutely injured and regenerated (at 3-5 weeks of age).
   b. Images of TA muscle sections from *mdx* mice at 5 weeks of age fluorescently stained with an anti-laminin-2α antibody are shown (the lower panels show enlarged images of the upper panels). Laminin-2α is a protein found in the basement membrane of myofibers so that the size of each myofiber can be observed by staining with an anti-laminin-2α antibody.
   c. The proportion of centrally nucleated myofibers (regenerated myofibers) in TA muscles of *mdx* mice at 5 weeks of age is shown (left bar: control group, right bar: G-CSF-treated group). In the G-CSF-treated group, muscle regeneration was significantly promoted.
   d. Immunostainings with RNMy2/9D2, laminin and DAPI at 4 weeks of age are shown (upper panel: control group, lower panel: G-CSF-treated group).
   e. The proportion of regenerating myocytes is shown as the proportion of RNMy2/9D2-positive cells in TA muscles at 4 weeks of age (left bar: control group, right bar: G-CSF-treated group).
   f. The distribution of the cross sectional areas of myofibers in TA muscles of *mdx* mice at 5 weeks of age is shown (left bars: control group, right bars: G-CSF-treated group). The proportion of small myofibers (regenerated myofibers) significantly increased in the G-CSF-treated group.
   g. The perimeters of TA muscles of *mdx* mice at 5 weeks of age are shown (left bar: control group, right bar: G-CSF-treated group). A significant increase was observed in the G-CSF-treated group.
   h. The results of an exercise tolerance test in *mdx* mice at 5 weeks of age (the distribution of the number of individuals vs. tolerance time) are shown (left bars: control group, right bars: G-CSF-treated group). Improvements in tolerance were observed in the G-CSF-treated group.
   i., j. The results of measurements of tetanic force (i) and specific force (j) in extensor digitorum longus muscles of *mdx* mice at 5 weeks of age are shown (left bars: control group, right bars: G-CSF-treated group).
[Figure 3]
   a. The survival curves of *csf3r*^{+/-} mice, *mdx* mice, and *mdx*/*csf3r*^{+/*-*} are shown. In *mdx*/*csf3r*^{+/-} mice, a biphasic death response was observed immediately after birth and at postnatal week 3-5 (the stage during which muscles are acutely degenerated and regenerated).
   b., c. The photographs (b) and body weights (c) of *csf3r*^{+/-} mice, *mdx* mice, and *mdx*/*csf3r*^{+/-}mice at postnatal day 2 are shown. As compared with *mdx* mice, *mdx*/*csf3r*^{+/-} had significantly lower body weights.
   d. Images of diaphragms of *csf3r*^{+/-} mice, *mdx* mice, and *mdx*/*csf3r*^{+/-} mice at postnatal day 2 stained with Evans blue are shown. In *mdx*/*csf3r*^{+/-} mice, necrosis and degeneration of diaphragms were observed.
   e. Images of diaphragms of *csf3r*^{+/-} mice, *mdx* mice, and *mdx*/*csf3r*^{+/-} mice at postnatal day 2 stained with hematoxylin and eosin (H&E stained) are shown. In *mdx*/*csf3r*^{+/-} mice, necrosis and degeneration of diaphragms were observed.
   f. The thicknesses of diaphragms of *csf3r*^{+/*-*} mice, *mdx* mice, and *mdx*/*csf3r*^{+/-} mice are shown. g. The body weight curves of *csf3r*^{+/-} mice and *mdx*/*csf3r*^{+/-} mice are shown.
[Figure 4]
   a. The gross appearances of *mdx* mice, *mdx*/*csf3r*^{+/-} mice and *mdx*/*csf3r*^{-/-} mice at postnatal day 7, and the gross appearances of *mdx*/*csf3r*^{+/-} mice and *mdx*/*csf3r*^{-/-} mice at postnatal day 13 are shown.
   b. H&E stainings of TA muscles of *mdx*/*csf3r*^{+/-} mice at 2-8 weeks of age are shown (lower panels: low magnification; upper panels: high magnification).
   c. The number of regenerated myocytes is shown as the number of centrally nucleated myocytes in TA muscles of *mdx* mice and *mdx*/*csf3r*^{+/-} mice at 5 weeks of age.
   d. The perimeters of TA muscles of *mdx* mice and *mdx*/*csf3r*^{+/-} mice at 5 weeks of age are shown.
   e. The tetanic forces determined ex vivo in TA muscles of *mdx* mice and *mdx*/*csf3r*^{+/-} mice at 5 weeks of age are shown.
   f. The specific forces determined ex vivo in TA muscles of *mdx* mice and *mdx*/*csf3r*^{+/-} mice at 5 weeks of age are shown.
[Figure 5]
   a. The gross appearances of *mdx* mice (mdx) and *dystrophin*/*utrophin* double knockout (mdx/utrn-/-) mice are shown. As compared with *mdx* mice, *mdx*/*utrn*^{-/-} mice show a significantly smaller body size and a considerably curved spine.
   b. Images of H&E stained TA muscle sections from *mdx*/*utrn*^{-/-} mice at 3-12 weeks of age are shown. More acute muscle injury and regeneration are observed as compared with *mdx* mice (Figure 1b).
   c. The total number of myocytes in TA muscle sections from *mdx*/*utrn*^{-/-} mice is shown.
   d. The proportion of centrally nucleated cells in TA muscles of *mdx*/*utrn*^{-/-} mice is shown.
   e. Images of TA muscle sections from *mdx*/*utrn*^{-/-} mice fluorescently stained with an anti-G-CSFR antibody, an anti-laminin-2α antibody and DAPI, respectively, and an image formed by superimposing these images (MERGE), and an enlarged image of the latter image are shown. More remarkable expression of G-CSFR in regenerating muscles is observed as compared with *mdx* mice (Figure 1c).
[Figure 6]
   a. A schedule of G-CSF administration to *mdx*/*utrn*^{-/-} mice is shown. G-CSF is administered at the stage during which muscles are acutely injured and regenerated (at 3-5 weeks of age).
   b. H&E stainings of TA muscle sections from *mdx*/*utrn*^{*-*/*-*} mice are shown (left panel: control group, right panel: G-CSF-treated group).
   c. The perimeters of TA muscles of *mdx*/*utrn*^{-/-} mice are shown (left bar: control group, right bar: G-CSF-treated group).
   d. The ratio of muscle wet weight to body weight of *mdx*/*utrn*^{-/-} mice is shown (TA: tibialis anterior muscle, Gas: gastrocnemius muscle, Quad: quadriceps muscle) (left bars: control group, right bars: G-CSF-treated group). In the G-CSF-treated group, a significant increase in gastrocnemius muscles and quadriceps muscles was found.
   e. The number of regenerated myocytes is shown as the number of centrally nucleated myocytes in TA muscles of *mdx*/*utrn*^{-/-} mice (left bar: control group, right bar: G-CSF-treated group).
   f. The number of myofibers in TA muscles of *mdx*/*utrn*^{-/-} mice is shown (left bar: control group, right bar: G-CSF-treated group).
   g. The distribution of the cross sectional areas of myofibers in TA muscles of *mdx*/*utrn*^{-/-} mice is shown (left bars: control group, right bars: G-CSF-treated group).
   h. The tetanic forces determined ex vivo in TA muscles of *mdx*/*utrn*^{-/-} mice are shown (left bar: control group, right bar: G-CSF-treated group).
   i. The specific forces determined ex vivo in TA muscles of *mdx*/*utrn*^{-/-} mice are shown (left bar: control group, right bar: G-CSF-treated group).
   j. The Kaplan-Meyer survival curves of *mdx*/*utrn*^{-/-} mice when G-CSF was administrated or not are shown.
   k. The tidal volumes per body weight at 8 weeks of age are shown (left bar: control group, right bar: G-CSF-treated group).

### DESCRIPTION OF EMBODIMENTS

Skeletal muscles are composed of long thin cells called myofibers and connective tissue filling the gaps between the cells to hold them together. The myofibers are each one cell called myocyte. The myocyte is a multinuclear cell having multiple nuclei. An assembly of myofibers forms a muscle bundle, and an assembly of muscle bundles forms a skeletal muscle.

As used herein, the treatment of a disease associated with skeletal muscle degeneration refers to any of inhibiting degeneration of muscles and promoting their regeneration; inhibiting the progression of muscle weakness; inhibiting myofiber disruption; increasing the ratio of regenerated myofibers; increasing muscles; or enhancing the muscle's ability to move.

The disease associated with skeletal muscle degeneration is not specifically limited, but typically muscular dystrophy including Duchenne, Becker, limb-girdle, congenital, distal, facioscapulohumeral and myotonic types. The therapeutic agents of the present invention are especially useful for Duchenne muscular dystrophy (DMD).

The therapeutic agents for diseases associated with skeletal muscle degeneration of the present invention comprise a granulocyte colony-stimulating factor (G-CSF) as an active ingredient.

The G-CSF used as an active ingredient of the therapeutic agents for diseases associated with skeletal muscle degeneration of the present invention can be any G-CSF, but preferably a highly purified G-CSF, more specifically having biological activities substantially identical to those of mammalian G-CSFs, especially human G-CSF. The source from which the G-CSF is derived is not specifically limited, and naturally derived or genetically engineered G-CSFs or the like can be used, preferably genetically engineered G-CSFs. The genetically engineered G-CSFs may have an amino acid sequence identical to those of naturally derived G-CSFs or may have a variant of such an amino acid sequence in which one or more amino acids are modified by deletion, substitution, addition or the like while retaining similar biological activities to those of naturally derived G-CSFs. The amino acid deletion, substitution, addition or the like can be made by a method known to those skilled in the art. For example, a polypeptide functionally comparable to a G-CSF can be prepared by those skilled in the art by introducing an amino acid variation into the G-CSF as appropriate using site-directed mutagenesis (Gotoh, T. et al. (1995) Gene 152, 271-275; Zoller, M. J. and Smith, M. (1983) Methods Enzymol. 100, 468-500; Kramer, W. et al. (1984) Nucleic Acids Res. 12, 9441-9456; Kramer, W. and Fritz, H. J. (1987) Methods Enzymol. 154, 350-367; Kunkel, T. A. (1985) Proc. Natl. Acad. Sci. USA. 82, 488-492; Kunkel (1988) Methods Enzymol. 85, 2763-2766) or the like. Amino acid variations may also occur in nature. Generally, an amino acid residue is preferably substituted by another amino acid whose side chain has a conserved property. For example, amino acids are classified by the properties of their side chains into hydrophobic amino acids (A, I, L, M, F, P, W, Y, V), hydrophilic amino acids (R, D, N, C, E, Q, G, H, K, S, T), amino acids having aliphatic side chains (G, A, V, L, I, P), amino acids having hydroxyl-containing side chains (S, T, Y), amino acids having sulfur-containing side chains (C, M), amino acids having carboxylate- and amide-containing side chains (D, N, E, Q), amino acids having base-containing side chains (R, K, H), and amino acids having aromatic-containing side chains (H, F, Y, W) (examples are shown by one-letter amino acid codes in each set of parentheses). It has already been known that polypeptides having a variant of an amino acid sequence in which one or more amino acid residues are deleted, added and/or substituted by another amino acid retain their biological activities (Mark, D. F. et al., Proc. Natl. Acad. Sci. USA (1984) 81, 5662-5666; Zoller, M. J. & Smith, M. Nucleic Acids Research (1982) 10, 6487-6500; Wang, A. et al., Science 224, 1431-1433; Daibadie-McFarland, G. et al., Proc. Natl. Acad. Sci. USA (1982) 79, 6409-6413).

Fusion proteins of a G-CSF with another protein can also be used. To create a fusion protein, the DNA encoding a G-CSF can be linked in-frame with the DNA encoding another protein and inserted into an expression vector and then expressed in a host, for example. The another protein to be fused with a G-CSF of the present invention is not specifically limited.

Chemically modified G-CSFs can also be used. Examples of chemically modified G-CSFs include, for example, G-CSFs manipulated to change the conformation of or add or remove a carbohydrate chain, G-CSFs conjugated to polyethylene glycol, vitamin B12 or a compound such as an inorganic or organic compound and the like.

The G-CSFs that can be used in the present invention may be those prepared by any process and include, for example, G-CSFs extracted and isolated/purified by various methods from cultures of a cell line of a human tumor cell or a human G-CSF-producing hybridoma or G-CSFs produced by genetic engineering techniques in E.coli, yeast, Chinese Hamster Ovary cells (CHO cells), C127 cells, COS cells, myeloma cells, BHK cells, insect cells or the like and extracted and isolated/purified by various methods. The G-CSFs used in the present invention are preferably G-CSFs prepared by genetic engineering techniques, preferably G-CSFs prepared by using mammalian cells (especially CHO cells) (e.g., see JPB H-1-44200, JPB H-2-5395, JPA S-62-129298, JPA S-62-132899, JPA S-62-236488, and JPA S-64-85098).

The therapeutic agents for diseases associated with skeletal muscle degeneration of the present invention can optionally contain suspending agents, solubilizers, stabilizers, isotonizing agents, preservatives, adsorption inhibitors, surfactants, diluents, excipients, pH modifiers, soothing agents, buffers, sulfur-containing reducing agents, antioxidants and the like as appropriate depending on the mode of administration or dosage form.

Examples of suspending agents include methylcellulose, Polysorbate 80, Polysorbate 20, hydroxyethylcellulose, gum acacia, gum tragacanth powder, sodium carboxymethylcellulose, polyoxyethylene sorbitan monolaurate, etc.

Solubilizers include polyoxyethylene hardened castor oil, Polysorbate 80, Polysorbate 20, nicotinic acid amide, macrogols, castor oil fatty acid ethyl esters, etc.

Stabilizers include dextran 40, methylcellulose, gelatin, sodium sulfite, sodium metasulfite, etc.

Isotonizing agents include e.g., D-mannitol, sorbitol, etc.

Preservatives include e.g., methyl paraoxybenzoate, ethyl paraoxybenzoate, sorbic acid, phenol, cresol, chlorocresol, etc.

Adsorption inhibitors include e.g., human serum albumin, lecithin, dextran, ethylene oxide/propylene oxide copolymers, hydroxypropyl cellulose, methylcellulose, polyoxyethylene hardened castor oil, polyethylene glycol, etc.

Typical examples of surfactants include:
nonionic surfactants, e.g., sorbitan fatty acid esters such as sorbitan monocaprylate, sorbitan monolaurate and sorbitan monopalmitate; glycerin fatty acid esters such as glycerin monocaprylate, glycerin monomyristate and glycerin monostearate; polyglycerin fatty acid esters such as decaglyceryl monostearate, decaglyceryl distearate and decaglyceryl monolinoleate; polyoxyethylene sorbitan fatty acid esters such as polyoxyethylene sorbitan monolaurate, polyoxyethylene sorbitan monooleate, polyoxyethylene sorbitan monostearate, polyoxyethylene sorbitan monopalmitate, polyoxyethylene sorbitan trioleate and polyoxyethylene sorbitan tristearate; polyoxyethylene sorbitol fatty acid esters such as polyoxyethylene sorbitol tetrastearate and polyoxyethylene sorbitol tetraoleate;
polyoxyethylene glycerin fatty acid esters such as polyoxyethylene glyceryl monostearate;
polyethylene glycol fatty acid esters such as polyethylene glycol distearate; polyoxyethylene alkyl ethers such as polyoxyethylene lauryl ether; polyoxyethylene polyoxypropylene alkyl ethers such as polyoxyethylene polyoxypropylene glycol ether, polyoxyethylene polyoxypropylene propyl ether and polyoxyethylene polyoxypropylene cetyl ether;
polyoxyethylene alkyl phenyl ethers such as polyoxyethylene nonyl phenyl ether;
polyoxyethylene hardened castor oils such as polyoxyethylene castor oil and polyoxyethylene hardened castor oil (polyoxyethylene hydrogenated castor oil); polyoxyethylene beeswax derivatives such as polyoxyethylene sorbitol beeswax; polyoxyethylene lanolin derivatives such as polyoxyethylene lanolin; and polyoxyethylene fatty acid amides such as polyoxyethylene stearic acid amide having an HLB of 6-18;
anionic surfactants, e.g., alkyl sulfates having a C10-18 alkyl group such as sodium cetyl sulfate, sodium lauryl sulfate and sodium oleyl sulfate; polyoxyethylene alkyl ether sulfates having an average number of moles of ethylene oxide added of 2-4 and a C10-18 alkyl group such as sodium polyoxyethylene lauryl sulfate; and alkyl sulfosuccinic acid ester salts having a C8-18 alkyl group such as sodium laurylsulfosuccinate; and
natural surfactants, e.g., lecithin; glycerophospholipids; sphingophospholipids such as sphingomyelin; and sucrose fatty acid esters of C12-18 fatty acids. Formulations of the present invention can contain one or more of these surfactants in combination. Preferred surfactants are polyoxyethylene sorbitan fatty acid esters such as Polysorbate 20, 40, 60 or 80, especially Polysorbates 20 and 80. Polyoxyethylene polyoxypropylene glycols such as poloxamers (e.g. Pluronic F-68®) are also preferred.

Sulfur-containing reducing agents include e.g., sulfhydryl-containing compounds such as N-acetylcysteine, N-acetylhomocysteine, thioctic acid, thiodiglycol, thioethanolamine, thioglycerol, thiosorbitol, thioglycolic acid and salts thereof, sodium thiosulfate, glutathione, and thioalkanoic acids having 1 to 7 carbon atoms.

Antioxidants include e.g., erythorbic acid, dibutylhydroxytoluene, butylhydroxyanisole, α-tocopherol, tocopherol acetate, L-ascorbic acid and salts thereof, L-ascorbyl palmitate, L-ascorbyl stearate, sodium bisulfite, sodium sulfite, triamyl gallate, propyl gallate or chelating agents such as disodium ethylenediamine tetraacetate (EDTA), sodium pyrophosphate and sodium metaphosphate.

Other components commonly added may also be contained, e.g., inorganic salts such as sodium chloride, potassium chloride, calcium chloride, sodium phosphate, potassium phosphate and sodium bicarbonate; and organic salts such as sodium citrate, potassium citrate and sodium acetate.

The therapeutic agents of the present invention can be administered in dosage forms suitable as injectables for local administration (e.g., subcutaneous, intracutaneous, intramuscular, etc.). Especially preferred local administration is intramuscular administration.

Injectables are prepared by dissolving the components described above in an aqueous buffer known in the field of solution formulations such as a phosphate buffer (preferably a sodium monohydrogen phosphate / sodium dihydrogen phosphate system) and/or a citrate buffer (preferably sodium citrate buffer) and/or an acetate buffer to prepare a solution formulation. The concentration of the buffer is normally 1-500 mM, preferably 5-100 mM, more preferably 10-20 mM. Injectables may be solution formulations or freeze-dried formulations.

The dose and the time and frequency of administration of the therapeutic agents for diseases associated with skeletal muscle degeneration containing a G-CSF as an active ingredient of the present invention can be determined as appropriate by those skilled in the art based on the pathological condition of the patient with a disease to be treated.

In order to obtain the inhibitory effect against muscle degeneration of the present invention, the therapeutic agents for diseases associated with skeletal muscle degeneration of the present invention are preferably administered at a dose of 0.1 to 500 *µ*g/kg/day, more preferably 1 to 50 *µ*g/kg/day when they are administered to humans.

However, the present invention is not limited by the G-CSF doses to humans.

The therapeutic agents for diseases associated with skeletal muscle degeneration of the present invention may also be used in combination with other drugs.

As shown in the following Examples, it was shown first that G-CSF is obviously effective for muscle regeneration and functional recovery in DMD model mice carrying a dystrophin mutation. Further, mice not only carrying a dystrophin mutation but also haploinsufficient for G-CSFR (abnormal expression of one copy of the gene) tend to die early, showing that G-CSF, which is a ligand for G-CSFR, is critical for inhibiting the progression of DMD. Moreover, G-CSF administration dramatically improved the muscle regeneration, functional recovery and survival of *dystrophin*/*utrophin* double knockout mice known as another type of DMD model mice showing severe symptoms similar to human DMD and resulting in early death. These results strongly suggest that G-CSF plays an important role in the skeletal muscle regeneration and survival of the DMD model mice and open the way to future clinical applications.

The present invention can be expected to be industrially applicable as therapeutic agents for muscular dystrophy against which any effective therapy has not existed so far.

The following Examples further illustrate the present invention without, however, limiting the scope of the invention thereto. Various changes and modifications can be made by those skilled in the art on the basis of the description herein, and such changes and modifications are also included in the present invention.

### EXAMPLES

The experimental methods used in the Examples are as follows.

### Animals

The *mdx* (C57/B6) mice and *utrophin* heterozygous (*utrophin*^{*+*/*-*}) mice were a kind gift from Dr. Shin'ichi Takeda (the National Center of Neurology and Psychiatry of Japan). *The csf3r*^{*-*/*-*} mice were a kind gift from Dr. Daniel C. Link (Washington University School of Medicine, St. Louis) (Richards, M. K., Liu, F., Iwasaki, H., Akashi, K. & Link, D. C. Pivotal role of granulocyte colony-stimulating factor in the development of progenitors in the common myeloid pathway. Blood 102, 3562-3568, doi:10.1182/blood-2003-02-0593 (2003)). All the experimental procedures and protocols were approved by the Institutional Animal Care and Use Committee at the Keio University and conformed to the NIH *Guide for the Care and Use of Laboratory Animals.* Knockout mice deficient in both *dystrophin* and *csf3r* were obtained by the following breeding program: male *csf3r* knockout mice were mated with female C57B/6 *mdx* mice (deficient in *dystrophin*) to produce F1 generation mice of all possible genotypes, i.e., heterozygous for *csf3r* (females and males) and either heterozygous for the *mdx* mutation (females) or hemizygous for the *mdx* mutation (males), and the resulting offspring were crossed among themselves. Knockout mice deficient in both *dystrophin* and *utrophin* were obtained by the following breeding program: male *utrophin* heterozygous mice were mated with female C57B/6 *mdx* mice to produce F1 generation mice heterozygous for *utrophin* (females and males) and either heterozygous for the *mdx* mutation (females) or hemizygous for the *mdx* mutation (males), and the resulting offspring were crossed among themselves (Deconinck, A. E. et al. Utrophin-Dystrophin-Deficient Mice as a Model for Duchenne Muscular Dystrophy. Cell 90, 717-727, doi:Doi: 10.1016/ s0092-8674 (00) 80532-2 (1997)).

### Genotyping

DNA was isolated from the tail of each mouse using the Easy-DNA ™ Kit (Invitrogen). A forward primer (5' CATAGTTATTAATGCATAGATATTCAG 3') complementary to the mouse dystrophin and a reverse primer (5' GTCACTCAGATAGTTGAAGCCATTTAG 3') complementary to the wild-type allele or a reverse primer (5'GTCACTCAGATAGTTGAAGCCATTTAA3') complementary to a mouse mutant dystrophin allele were used for PCR analysis to determine the mutant dystrophin allele. Both PCR reactions were performed under conditions of 95 °C, 4 min; then 34 cycles of 95 °C, 1 min, 55 °C, 1 min and 72 °C, 1 min followed by 72 °C, 10 min. Each reaction yielded a 275 bp product. All PCR reactions were performed by using the GoTaq DNA Polymerase kit (Promega, Madison, WI, USA). A forward primer (5' GTGAAGGATGTCATGAAAG 3') complementary to exon 7 of the mouse utrophin and a reverse primer (5' TGAAGTCCGAAAGAGATACC 3') complementary to intron 7 or a reverse primer (5' ACGAGACTAGTGAGACGTGC 3') complementary to the PGK promoter located within the Neo-knockout cassette were used for PCR analysis to determine the *utrophin*-knockout status. PCR reactions were performed under conditions of 35 cycles of 94 °C, 30 sec, 57 °C, 30 sec and 72 °C, 25 sec. A forward primer (5' AGTTCACCAGGCAGGTGAGT 3') complementary to the mouse csf3r and a reverse primer (5' GTAGGCCTAGTTCATACCTG 3') complementary to the wild-type allele or a reverse primer (5' TCCAGACTGCCTTGGGAAAA 3') complementary to a mouse mutant *csf3r* allele were used for PCR analysis to determine the mutant *csf3r* allele. Both PCR reactions were performed under conditions of 95 °C, 4 min; then 30 cycles of 95 °C, 30 sec, 60 °C, 30 sec and 72 °C, 45 sec. Each reaction yielded a 275 bp product. All PCR reactions were performed by using the Taq DNA Polymerase kit (TaKaRa, Japan).

### G-CSF treatment

G-CSF (Neutrogin™, 100 µg; Chugai Pharmaceutical Co., Ltd.) was diluted in 1 ml of saline. G-CSF was intraperitoneally administered to 3-week-old male mice at a dose of 5 µg/day for two weeks by using a 27-gauge needle. Mice of the G-CSF-treated group and control group were sacrificed at 5 weeks of age.

### Histological analysis

Frozen sections (10 µm) of tibialis anterior (TA) muscles were stained with hematoxylin and eosin (H&E stain). H&E staining was performed on the sections to determine the percentage of regenerated muscle. The fiber cross area (FSA) was determined on tibialis anterior sections (7 µm) immunofluorescently stained for laminin-2 alpha using software (BZ-HIC: KEYENE). Digital photographs of each section were taken with a digital camera (BIOREVO: KEYENCE) at a magnification of 20x. Evans blue purchased from Sigma was dissolved in phosphate buffered saline (PBS: 0.15 M NaCl, 10 mM phosphate buffer, pH 7.0). Using a 27-gauge needle, 0.05 mL of 1 % Evans blue was intraperitoneally administered at postnatal day 2. The mice were sacrificed 3 hours later.

### Immunofluorescence

All immunofluorescence assays were performed on sections of 7 µm. The samples were incubated with Triton X-100 for 5 min at room temperature, then washed, and incubated with the following primary antibodies: an anti-G-CSFR antibody (Santa Cruz Biotechnology (sc-9173), 1:50 dilution), an anti-laminin-2α antibody (Sigma (L0663), 1:1000 dilution), an anti-laminin antibody (L9393, 1:1000 dilution) and RNMy2/9D2 (ab49457, 1:30 dilution, abcam). After incubation overnight, bound antibodies were visualized with a secondary antibody conjugated to Alexa 488 or Alexa 546 (Molecular Probes, Minato-ku, Tokyo). Nuclei were stained with 4',6-diamidino-2-phenylindole dihydrochloride (DAPI; Invitrogen).

### Muscle force measurements

Extensor digitorum longus (EDL) muscles were isolated and removed from 5 week-old mice. The muscles were carefully mounted in a chamber filled with PSS buffer (95 % O₂, 5 % CO₂) and maintained at 30 °C. One muscle tendon was attached to a steel hook in the chamber, and the other was tied with 5-0 surgical silk to the lever arm of a dual-mode servomotor system (Electronic Stimulator; NIHON KOHDEN CORPORATION, Tokyo, Japan). The muscles were stretched to the length at which the maximum single twitch and tetanic forces were produced (optimal length; Lo). The corresponding tetanic force was then measured at 150 Hz, 5 sec duration for 20 minutes with a rest period of 120 seconds for changing the buffer. The muscles were adjusted to the optimum length (Lo) before all the measurements. For comparative purposes, all the measurements were normalized to the muscle cross-sectional area (CSA), which was calculated by dividing the muscle mass by the product of the length and the mammalian skeletal muscle density (1.056 mg/mm³). The specific force (N/cm²) was calculated with muscle density assumed as 1.056 mg/mm³.

### Exercise tolerance tests

Mice were subjected to an exhaustion treadmill test. Each mouse was placed on the belt of a one-lane motorized treadmill (MK-680S TREADMILL for RATS &MICE; Muromachi). The test was run at an inclination of 0° initially at 5 m/min for 5 minutes, and the speed was then increased by 1 m/min every minute. The endpoint of the test was when the mouse remained in the electrical stimulus-applying zone for 20 seconds or more.

### Whole-body plethysmography

Animals were placed in a chamber where they can move freely. The chamber was connected to a high-gain differential pressure transducer (Valydine MP45, Validyne, North Ridge, CA). As the animals breathed, changes in pressure were converted to signals representing tidal volume (TV), which were amplified (BMA 830; CWE, Ardmore, PA), recorded on a strip-chart recorder (Dash 10; Astro-Med, West Warwick, RI), stored in a computer with respiratory acquisition software for analysis. Oxygen consumption and carbon dioxide production were determined by the open-circuit method using Beckman OM-14 and LB-2 analyzers. The parameter TV was analyzed in real time to calculate the average 5 times every 2 minutes for 3 consecutive hours. TV was normalized to body weight.

### Statistical analysis

The data were analyzed using the StatView J-4.5 software. Values are reported as means±SD. Comparisons between groups were performed by one-way ANOVA. A Scheffe's F-test was used to determine the level of significance. The probability level accepted for significance was *P* < 0.05.

### Example 1: G-CSF receptor is expressed in regenerative myocytes in mdx mice.

The appearance of *mdx* mice was grossly normal in terms of body size, muscle size and body length (Figure 1a). It has been reported that patients with DMD show progressive physical impairment leading to death by the age of 20-30, whereas mature *mdx* mice exhibit little physical impairment and have a normal life span (Evans, N. P., Misyak, S. A., Robertson, J. L., Bassaganya-Riera, J. & Grange, R. W. Dysregulated intracellular signaling and inflammatory gene expression during initial disease onset in Duchenne muscular dystrophy. Am J Phys Med Rehabil 88, 502-522 (2009)).

However, pathological analyses show that muscle degeneration and regeneration evidently occurred in *mdx* mice between 3 and 5 weeks of age (Figure 1b). Until 2 weeks of age, little degeneration and regeneration occurred, but substantial amounts of degenerative muscles and accumulated leukocytes were observed at 3 weeks of age. At 5 weeks of age, varying compositions of small round and relatively large regenerative myocytes were found. At 8 weeks of age, degeneration calmed down. The total number of myocytes in TA decreased at 2 to 4 weeks of age, and then recovered (Figure 1c).

Generally, healthy myocytes are peripherally nucleated while centrally nucleated myocytes are regenerating or regenerated myocytes. In *mdx* mice, centrally nucleated myocytes were observed after 4 weeks of age and increased until 12 weeks of age (Figure 1d). These data indicate that myofibers are initially degenerated and then regenerated to nearly compensate for the degeneration.

To examine whether or not G-CSF signaling is involved in muscle degeneration in *mdx* mice, immunofluorescence staining for G-CSF receptor (G-CSFR) was performed. TA (tibialis anterior) muscle sections at different developing stages were observed to show that G-CSFR was highly expressed in a small round shape in centrally nucleated cells (regenerating muscles) (Figure 1e). Continuous immunofluorescence staining analysis shows that G-CSFR-expressing cells appeared most frequently at 3 weeks to 5 weeks of age, which is consistent with the regenerative stage in *mdx* mice. These results suggested that G-CSF is involved in muscle regeneration of *mdx* mice.

### Example 2: Influence of G-CSF on skeletal muscle function in mdx mice through skeletal muscle regeneration.

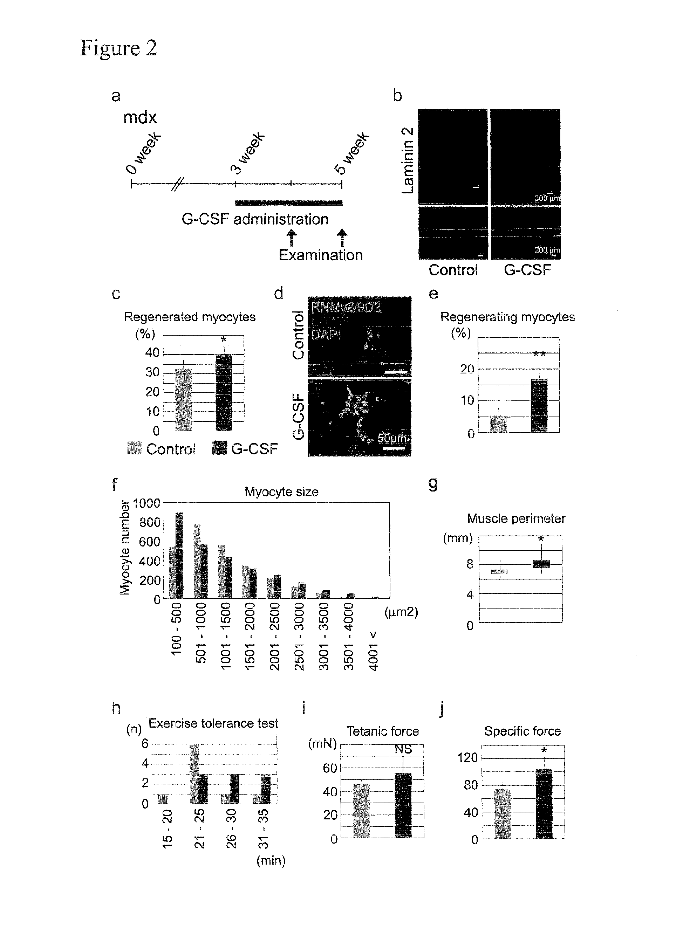

Based on the fact that G-CSFR-expressing cells appear most frequently at 3 weeks to 5 weeks of age in regenerating myocytes in *mdx* mice, we verified whether or not skeletal muscle regeneration and skeletal muscle function can be improved in *mdx* mice by administering G-CSF. G-CSF was intraperitoneally administered to *mdx* mice daily between 3 weeks and 5 weeks of age (Figure 2a) and examined gross appearance and functional differences. To show the morphology of each skeletal myocyte, we performed immunostaining for laminin 2α, which is the main isoform in the basement membrane of muscle (Patton, B. L., Miner, J. H., Chiu, A. Y. & Sanes, J. R. Distribution and Function of Laminins in the Neuromuscular System of Developing, Adult, and Mutant Mice. The Journal of Cell Biology 139, 1507-1521, doi:10.1083/jcb.139.6.1507 (1997)). Laminin 2α is a protein found in the basement membrane of myofibers so that the size of each myofiber can be observed by staining with laminin 2α. The results show that the number of centrally nucleated myofibers (regenerated myofibers) significantly increased at 5 weeks of age in the G-CSF-treated group as compared with the control group (Figure 2b, c).

RNMy2/9D2 recognizes a myosin heavy chain (MHC) present during the embryonic and neonatal period in the development of skeletal muscle and also occurring in newly formed adult regenerating myocytes (Bigard, et al. Changes in myosin heavy chain profile of mature regenerated muscle with endurance training in rat. Acta Physiologica Scandinavica 165, 185-192 (1999)). Immunostaining with RNMy2/9D2 at 4 weeks of age showed that G-CSF significantly increased the number of regenerating myocytes (Figure 2d, e). The individual cross sectional areas of myofibers at 5 weeks of age show a substantially increased proportion of small myofibers (regenerated myofibers), which means a significant increase of newly formed regenerated skeletal muscle cells (Figure 2f). These results suggest that G-CSF increases the number of regenerating myofibers. Because muscle size is one of predictive values for muscle function, the muscle perimeter and muscle wet weight were determined to show that the perimeter of TA muscle at 5 weeks of age was significantly increased by G-CSF administration (Figure 2g, left bar: control group, right bar: G-CSF-treated group).

Muscle weakness is one of the most prominent features of muscular dystrophy (Bushby, K. et al. Diagnosis and management of Duchenne muscular dystrophy, part 1: diagnosis, and pharmacological and psychosocial management. The Lancet Neurology 9, 77-93, doi:Doi: 10.1016/s1474-4422 (09) 70271-6 (2010)). Thus, an exercise tolerance test was performed to evaluate in vivo muscle function. The results showed that G-CSF treatment significantly improved exercise tolerance at 5 weeks of age (Figure 2h, the distribution of the number of individuals vs. tolerance time) (left bars: control group, right bars: G-CSF-treated group).

Moreover, tetanic force (Figure 2i) and specific force (Figure 2j) analyses in extensor digitorum longus (EDL) muscles of *mdx* mice also showed that G-CSF administration significantly improved ex vivo muscle-specific functions at 5 weeks of age (Figure 2i, j) (left bars: control group, right bars: G-CSF-treated group).

These data indicate that G-CSF significantly improves muscle function in *mdx* mice through regeneration.

### Example 3: The role of G-CSF signaling in the survival of muscular dystrophy model mice through diaphragm degeneration.

To clarify the roles of G-CSF and G-CSFR in muscular dystrophy, G-CSFR-knockout *(csf3r*^{-/-}*)* mice were used. The life span of the *csf3r*^{*-*/-} mice was normal though they showed a mild hematological disorder.

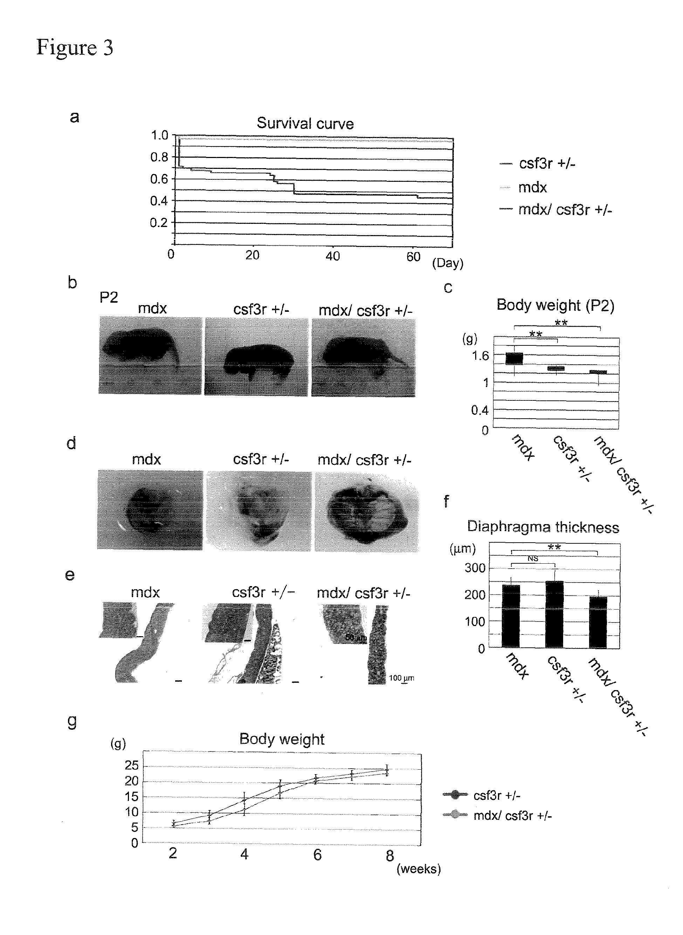

We hypothesized that G-CSFR homozygous knockout mice further carrying an *mdx* mutation (*mdx*/*csf3r*^{*-*/-}) would show severe injury in muscle function, but surprisingly, a biphasic death response was observed immediately after birth and at postnatal week 3 to 5 (the stage during which muscles are acutely degenerated/regenerated) even in G-CSF heterozygous *mdx* (*mdx*/*csf3r*^{+/-}) mice (Figure 3a). The surprising fact that death was observed in *mdx*/*csf3r*^{+/-} mice indicates the importance of G-CSF signaling in muscular dystrophy. Neither *csf3r*^{+/-} nor *mdx*/*csf3r*^{+/-} mice differed in gross appearance from *mdx* mice (Figure 3b). However, the body weights of *csf3r*^{+/-} mice and *mdx*/*csf3r*^{+/-} mice at day 2 significantly decreased as compared with *mdx* mice (Figure 3c).

To elucidate the cause of early lethality, we observed the gross appearances of diaphragms and lesions. The diaphragms of *csf3r*^{+/-} mice, *mdx* mice, and *mdx*/*csf3r*^{+/-} mice immediately after birth were stained with Evans blue. In *mdx*/*csf3r*^{+/-} mice, necrosis and degeneration of diaphragms were evidently observed in contrast to *mdx* mice and *csf3r*^{+/-} mice, both of which showed almost no degenerative lesion in their diaphragms (Figure 3d, e). The thickness of diaphragms was significantly smaller in *mdx*/*csf3r*^{+/-} mice (Figure 3e, f). The average body weight of the remaining survivors was consistently lower in *mdx*/*csf3r*^{+/-} mice than *csf3r*^{+/-} mice (Figure 3g). It should be noted that the difference between *csf3r*^{+/-} mice and *mdx*/*csf3r*^{+/-} mice apparently decreases on and after 5 weeks of age on the graph because *mdx*/*csf3r*^{+/-} mice having a strong phenotype die by the age of 5 weeks while relatively healthy *mdx*/*csf3r*^{+/-} mice survive.

### Example 4: The role of G-CSF in the late survival of muscular dystrophy model mice.

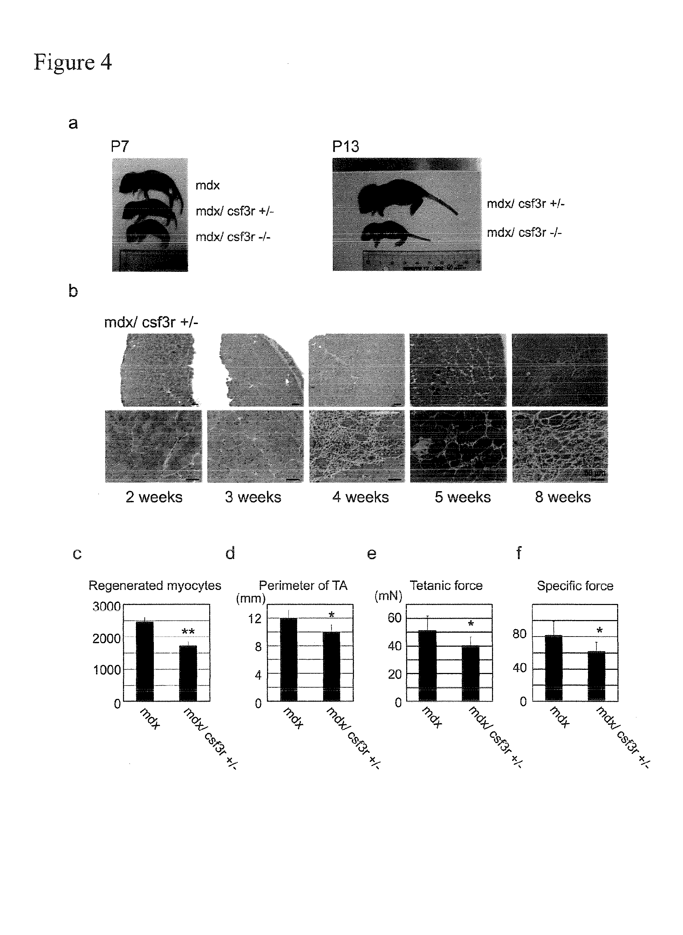

In *mdx* mice, skeletal muscle degeneration and regeneration was prominent at around 3 to 5 weeks. Thus, we considered that muscle regeneration and muscle function would be further worsened in *mdx*/*csf3r*^{+/-} mice and *mdx*/*csf3r*^{-/-} mice. A comparison of the gross appearances of *mdx* mice, *mdx*/*csf3r*^{+/-} mice and *mdx*/*csf3r*^{*-*/-} mice shows that *mdx*/*csf3r*^{+/-} mice and *mdx*/*csf3r*^{-/-} mice were smaller than *mdx* mice in body size at postnatal day 7 and that *mdx*/*csf3r*^{-/-} mice were evidently smaller at postnatal day 13 (Figure 4a). In *mdx*/*csf3r*^{+/-} mice that survived past the lethal period immediately after birth, regenerating myocytes decreased and degeneration considerably proceeded at around 4 weeks as well as 8 weeks later (Figure 4b). The number of regenerated myocytes significantly decreased in *mdx*/*csf3r*^{+/-} mice at 5 weeks (Figure 4c). Muscle size also significantly decreased in *mdx*/*csf3r*^{+/-} mice (Figure 4d). Ex vivo muscle tension examination also showed a decrease in tetanic force (Figure 4e) and specific force (Figure 4f) in *mdx*/*csf3r*^{+/-} mice. These results suggest that G-CSF is critical for skeletal muscle regeneration in *mdx* mice.

### Example 5: G-CSFR is expressed in regenerative myocytes in severe muscular dystrophy model mice.

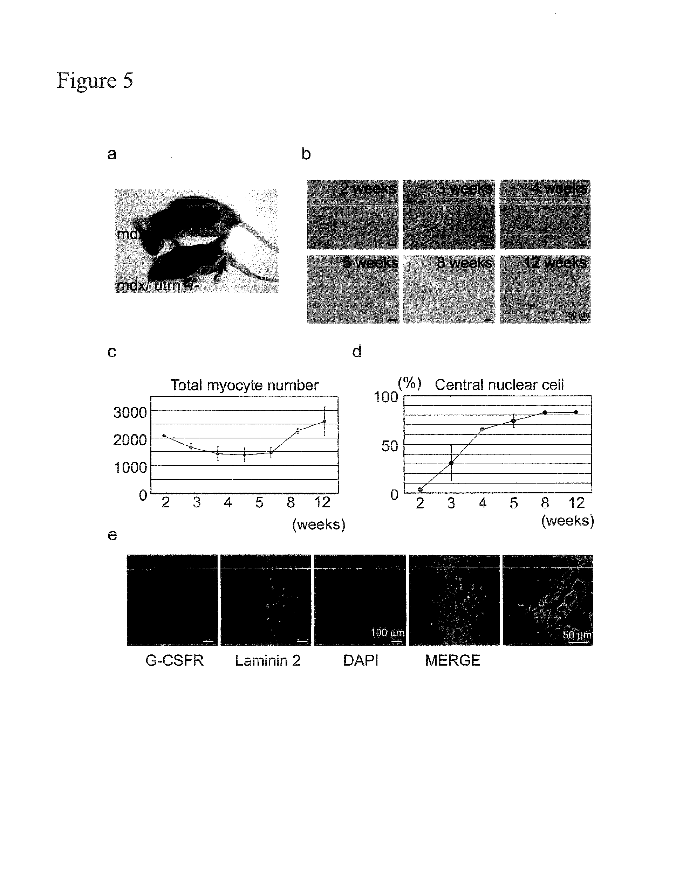

The absence of dystrophin in the myocyte membrane leads to the onset of Duchenne muscular dystrophy (DMD) in humans, which induces severe muscle weakness and muscular atrophy, resulting in death at a young age. However, *dystrophin-deficient mdx* mice appear physically normal despite of potential muscle degeneration and regeneration. Utrophin is a dystrophin-related protein sharing functional motifs with dystrophin. *Mdx*/*utrn*^{*-*/-} mice that express neither *dystrophin* nor its homolog *utrophin* show severe dystrophy similar to human DMD patients so that they can be an animal model of DMD (Grady, R. M. et al. Skeletal and Cardiac Myopathies in Mice Lacking Utrophin and Dystrophin: A Model for Duchenne Muscular Dystrophy. Cell 90, 729-738, doi:Doi: 10.1016/s 0092-8674 (00) 80533-4 (1997); Deconinck, A. E. et al. Utrophin-Dystrophin-Deficient Mice as a Model for Duchenne Muscular Dystrophy. Cell 90, 717-727, doi:Doi: 10.1016/s 0092-8674 (00) 80532-2 (1997)). The *mdx*/*utrn*^{-/-} mice show significantly small body size and abnormally curved spine as compared with *mdx* mice (Figure 5a).

H&E stainings of TA muscle sections at 2-12 weeks of age show that muscle injury occurred more acutely in *mdx*/*utrn*^{*-*/-} mice at 3 weeks / 4 weeks / 5 weeks of age as compared with *mdx* mice (Figure 1b) and that this injury was persistent later (Figure 5b).

The total number of myocytes in TA muscles decreased at 2-5 weeks and then recovered (Figure 5c). In *mdx*/*utrn*^{-/-} mice, centrally nucleated myocytes were observed after 3 weeks (Figure 5d).

Images of TA sections from *mdx*/*utrn*^{*-*/-} mice fluorescently stained with an anti-G-CSFR antibody, an anti-laminin-2α antibody and DAPI, respectively, and an image formed by superimposing these images (MERGE), and an enlarged image of the latter are shown (Figure 5e). More remarkable expression of G-CSFR in regenerating muscles is observed as compared with *mdx* mice (Figure 1e).

### Example 6: G-CSF improves skeletal muscle regeneration and function in severe muscular dystrophy model mice.

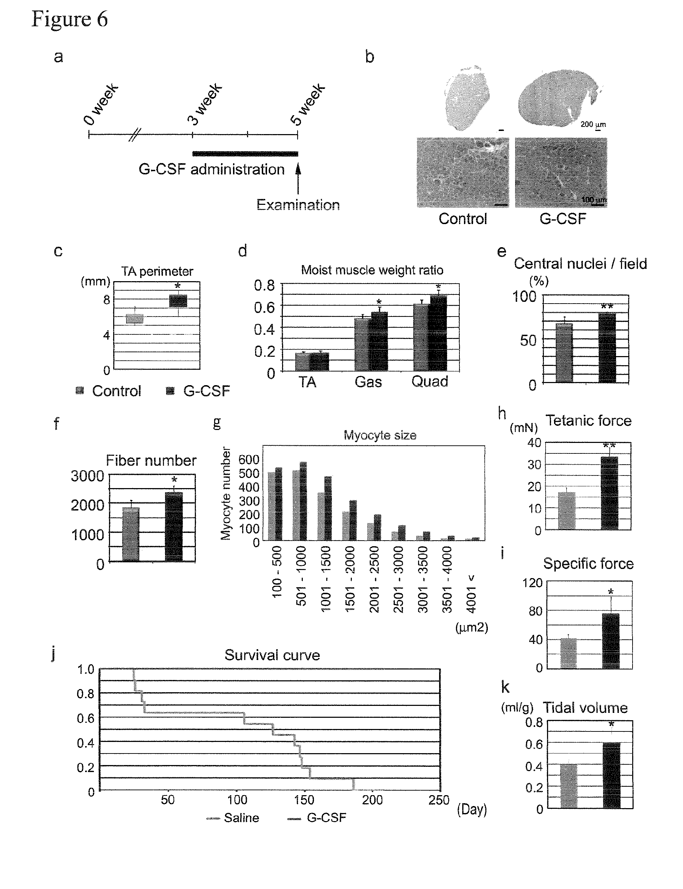

G-CSF was administered to *mdx*/*utrn*^{-/-} mice according to the administration schedule described in Figure 6a. Specifically, G-CSF was intraperitoneally administered once daily at the stage during which muscles were acutely injured and regenerated (at 3-5 weeks of age).

Photographs of H&E stained TA muscle sections (Figure 6b), and the perimeters of TA muscles (Figure 6c) are shown (left bar: control group, right bar: G-CSF-treated group). It is shown that muscle size was increased by G-CSF administration. Moreover, the ratio of muscle wet weight to body weight tended to increase in tibialis anterior muscle and significantly increased in gastrocnemius muscle (Gastrocnemius) and quadriceps muscle (Quadriceps) by G-CSF administration (Figure 6d) (TA: tibialis anterior muscle, Gas: gastrocnemius muscle, Quad: quadriceps muscle) (left bars: control group, right bars: G-CSF-treated group).

The proportion of centrally nucleated myofibers (regenerated myofibers) (Figure 6e) and the number of myofibers (Figure 6f) (left bar: control group, right bar: G-CSF-treated group) in tibialis anterior muscles of *mdx*/*utrn*^{-/-} mice at 3 to 5 weeks of age are shown. G-CSF administration increased the proportion of regenerated myofibers and the number of myofibers. The distribution of the cross sectional areas of myofibers in TA muscle is shown (Figure 6g) (left bars: control group, right bars: G-CSF-treated group). In the G-CSF-treated group, the sizes of all myocytes significantly increased. Ex vivo muscle tension examination also shows that G-CSF administration significantly improved the tetanic force (Figure 6h) and the specific force (Figure 6i). These results indicate that G-CSF improves skeletal muscle regeneration and muscle function in *mdx*/*utrn*^{-/-} mice.

Next, we tested whether or not long term G-CSF administration could contribute to the survival of *mdx*/*utrn*^{-/-} mice. The *mdx*/*utrn*^{-/-} mice started to die from 3 weeks of age and all died by the age of 180 days (Figure 6j). The G-CSF-treated group received G-CSF daily between days 3 and 5 and then twice a week. Surprisingly, all *mdx*/*utrn* ^{-/-}mice were found to survive up to 200 days by G-CSF administration (Figure 6j). The results of whole-body plethysmography examination indicated that G-CSF administration significantly increases respiratory function including tidal volume in *mdx*/*utrn*^{*-*/-} mice at 8 weeks (Figure 6k). Moreover, *mdx*/*utrn*^{-/-} mice treated with G-CSF show improved physical activity at 150 days. G-CSF treatment did not affect cardiac morphology known as a marker of cardiomyopathy that is one of fatal phenotypes of DMD (data not shown). These data strongly suggest that G-CSF improves skeletal muscle regeneration and saves the life of DMD model in mice.

## Claims

1. A therapeutic agent for a disease associated with skeletal muscle degeneration comprising a granulocyte colony-stimulating factor (G-CSF) as an active ingredient.

2. The therapeutic agent of claim 1 wherein the disease associated with skeletal muscle degeneration is muscular dystrophy.

3. The therapeutic agent of claim 2 wherein the disease associated with skeletal muscle degeneration is Duchenne muscular dystrophy.
